# EUROPEAN PATENT SPECIFICATION

(11) **EP 1 461 437 B1**
(45) Date of publication and mention of the grant of the patent: **07.11.2007**
(21) Application number: 02788244.8
(22) Date of filing: 23.12.2002
(51) Int. Cl.: C12N 15/63, C12N 15/67, C12N 15/70

(54) **EXPRESSION VECTORS AND PROMOTERS FOR HETEROLOGOUS GENE EXPRESSION**
EXPRESSIONSVEKTOREN UND PROMOTOREN FÜR HETEROLOGE GENEXPRESSION
VECTEURS D'EXPRESSION ET PROMOTEURS POUR L'EXPRESSION DE GENES HETEROLOGUES

(30) Priority: 21.12.2001 PT 10270401
(43) Date of publication of application: 29.09.2004
(73) Proprietor: Biotecnol S.A., 2741-901 Porto Salvo (PT)
(72) Inventor: KELLY, Andrew, Graham, Biotecnol SA, P-2780-920 Oeiras (PT); FILIPE, Susana, Melquiades, Biotecnol SA, P-2780-920 Oeiras (PT); GNADT, Nicole, Claudia, Biotecnol SA, P-2780-920 Oeiras (PT); BARBAS, Ana, Lucia, Biotecnol SA, P-2780-920 Oeiras (PT); PISSARRA, Pedro de Noronha Biotecnol SA, P-2780-920 Oeiras (PT)
(74) Representative: Cornish, Kristina Victoria Joy
(86) International application number: PCT/GB2002/005888
(87) International publication number: WO 2003/056020

(56) References cited:
- SEEGER C ET AL: "Identification and characterization of genes (xapA, xapB, and xapR) involved in xanthosine catabolism in Escherichia coli." JOURNAL OF BACTERIOLOGY. UNITED STATES OCT 1995, vol. 177, no. 19, October 1995 (1995-10), pages 5506-5516, XP002240493 ISSN: 0021-9193
- LIN CHING-SHWUN ET AL: "Regulation of human PDE5A2 intronic promoter by cAMP and cGMP: Identification of a critical Sp1-binding site." BIOCHEMICAL AND BIOPHYSICAL RESEARCH COMMUNICATIONS, vol. 280, no. 3, 26 January 2001 (2001-01-26), pages 693-699, XP002199272 ISSN: 0006-291X
- HEATON JOANNE H ET AL: "Cyclic nucleotide regulation of type-1 plasminogen activator-inhibitor mRNA stability in rat hepatoma cells: Identification of cis-acting sequences." JOURNAL OF BIOLOGICAL CHEMISTRY, vol. 273, no. 23, 5 June 1998 (1998-06-05), pages 14261-14268, XP002240494 ISSN: 0021-9258
- DATABASE BIOSIS [Online] BIOSCIENCES INFORMATION SERVICE, PHILADELPHIA, PA, US; 1989 MIRONOV A.S., PECHAEVA G.D. AND SUKHODOLETS V.V.: "Interaction of negative CYTR and positive cAMP-CRP control in the promoter region of the uridine phosphorylase UDP gene in Escherichia coli K-12" Database accession no. PREV198988037406 XP002247779 & GENETIKA, vol. 25, no. 3, 1989, pages 438-447, XP009013585
- DATABASE BIOSIS [Online] BIOSCIENCES INFORMATION SERVICE, PHILADELPHIA, PA, US; VEIKO V.P., SIPRASHVILI Z.Z. ET AL.: "Cloning and expression in Escherichia coli of thymidine phosphorylase under the control of the promoter of uridine phosphorylase." Database accession no. PREV199598047856 XP002247780 & BIOTEKHNOLOGIYA, no. 4, 1994, pages 2-4, XP009013583

### SEQUENCE LISTING

<110> Biotecnol S.A.
<120> vectors and promoters for gene expression
<130> -
<140> PCT/GB2002/005888
   <141> 2002-12-23
<150> PT102704
   <151> 2001-12-21
<160> 20
<170> PatentIn version 3.2
<210> 1
   <211> 29
   <212> DNA
   <213> Artificial Sequence
<220>
   <223> PCR oligonucleotide primer
<400> 1
   acggtacctt ttgctatctg cgatttgcg 29
<210> 2
   <211> 33
   <212> DNA
   <213> Artificial Sequence
<220>
   <223> PCR oligonucleotide primer
<400> 2
   ctcattaaaa ggatccgcgg ctctgctctt cag 33
<210> 3
   <211> 30
   <212> DNA
   <213> Artificial sequence
<220>
   <223> PCR oligonucleotide primer
<400> 3
   ccaagcttag cataattccc tatgccgatc 30
<210> 4
   <211> 26
   <212> DNA
   <213> Artificial Sequence
<220>
   <223> PCR oligonucleotide primer
<400> 4
   gaacctgaga catatgtatc cttttg 26
<210> 5
   <211> 31
   <212> DNA
   <213> Artificial Sequence
<220>
   <223> PCR oligonucleotide primer
<400> 5
   gtggtcacca tgggtatcct ttttctgtag g 31
<210> 6
   <211> 29
   <212> DNA
   <213> Artificial Sequence
<220>
   <223> PCR oligonucleotide primer
<400> 6
   atatgggccc atggatcccg tcgttttac 29
<210> 7
   <211> 29
   <212> DNA
   <213> Artificial Sequence
<220>
   <223> PCR oligonucleotide primer
<400> 7
   agtgtgaagc ttattatttt tgacaccag 29
<210> 8
   <211> 35
   <212> DNA
   <213> Artificial Sequence
<220>
   <223> PCR oligonucleotide primer
<400> 8
   aagaatccca tatgttccca accattccct tatcc 35
<210> 9
   <211> 38
   <212> DNA
   <213> Artificial Sequence
<220>
   <223> PCR oligonucleotide primer
<400> 9
   cgcggatcca agcttattag aagccacagc tgccctcc 38
<210> 10
   <211> 32
   <212> DNA
   <213> Artificial Sequence
<220>
   <223> PCR oligonucleotide primer
<400> 10
   aagaatccca tatgcacaag tgcgatatca cc 32
<210> 11
   <211> 38
   <212> DNA
   <213> Artificial sequence
<220>
   <223> PCR oligonucleotide primer
<400> 11
   aaggatccca agcttagctc gaacactttg aatatttc 38
<210> 12
   <211> 35
   <212> DNA
   <213> Artificial Sequence
<220>
   <223> PCR oligonucleotide primer
<400> 12
   cctacagaaa aaggaggtac ccatggatcc cgtcg 35
<210> 13
   <211> 36
   <212> DNA
   <213> Artificial Sequence
<220>
   <223> PCR oligonucleotide primer
<400> 13
   cgggatccat gggtacctcc tttttctgta gggtgg 36
<210> 14
   <211> 47
   <212> DNA
   <213> Artificial Sequence
<220>
   <223> PCR oligonucleotide primer
<400> 14
   ccctacagaa aaaggagata tcccatggat cccgtcgttt tacaacg 47
<210> 15
   <211> 49
   <212> DNA
   <213> Artificial sequence
<220>
   <223> PCR oligonucleotide primer
<400> 15
   cgacgggatc catgggatat ctcctttttc tgtagggtgg aatctaacg 49
<210> 16
   <211> 1232
   <212> DNA
   <213> Escherichia coli
<220>
   <221> CDS
   <222> (241)..(1140)
<400> 16
   tcattaaaag gatccgcggc tctgctcttc agagctgctt ttatgataaa ggttaattag      60
   tgaaagatat tttattttct tccggcgttg gttttggtat tggtgctttg tttacaatcg     120
   tgcgcttgcc tattcctgta ccaaatgtat taccgggtat attatccatt gtgtttatgt     180
   atgtcggata tctggtggtg aaatacttta tgccatgata atttaatacg atgtatttat     240
   tat atg gag cac tta att atg gaa cgc gta tac aga aca gat ctt aag       288
   Tyr Met Glu His Leu Ile Met Glu Arg Val Tyr Arg Thr Asp Leu Lys
   1               5                   10                  15
   ttg ctc cgt tat ttt ctt gcc gta gcg gaa gag ttg cat ttt ggc cgc       336
   Leu Leu Arg Tyr Phe Leu Ala Val Ala Glu Glu Leu His Phe Gly Arg
               20                  25                  30
   gca gca gcg cgt tta aat atg tct cag cct ccg ctc agc att cat att       384
   Ala Ala Ala Arg Leu Asn Met Ser Gln Pro Pro Leu Ser Ile His Ile
           35                  40                  45
   aaa gag ctg gaa aat caa ctc ggc acg cag ctt ttt att cgc cat tcg       432
   Lys Glu Leu Glu Asn Gln Leu Gly Thr Gln Leu Phe Ile Arg His Ser
       50                  55                  60
   cgc agc gtc gta ctg aca cac gcg ggc aaa atc ttg atg gaa gaa tcg       480
   Arg Ser Val Val Leu Thr His Ala Gly Lys Ile Leu Met Glu Glu Ser
   65                  70                  75                  80
   cgt cga ttg ctg gtg aat gct aat aat gta ttg gct cgg att gaa caa       528
   Arg Arg Leu Leu Val Asn Ala Asn Asn Val Leu Ala Arg Ile Glu Gln
                   85                  90                  95
   ata ggt cgg gga gaa gca ggg cgg att gaa ctc ggc gta gtg gga acg       576
   Ile Gly Arg Gly Glu Ala Gly Arg Ile Glu Leu Gly Val Val Gly Thr
               100                 105                 110
   gca atg tgg gga cgg atg cgc ccg gtt atg cgg cga ttc ctc agg gaa       624
   Ala Met Trp Gly Arg Met Arg Pro Val Met Arg Arg Phe Leu Arg Glu
           115                 120                 125
   aat cct aac gtt gac gtt ctt ttt cgc gaa aag atg ccc gcg atg caa       672
   Asn Pro Asn Val Asp Val Leu Phe Arg Glu Lys Met Pro Ala Met Gln
       130                 135                 140
   atg gcc ttg ctg gaa cgc cgc gaa ctt gat gcc ggg atc tgg cga atg         720
   Met Ala Leu Leu Glu Arg Arg Glu Leu Asp Ala Gly Ile Trp Arg Met
   145                     150                 155                 160
   gcg aca gaa cca ccg act ggt ttt acc agc tta cgg ttg cat gaa tcg         768
   Ala Thr Glu Pro Pro Thr Gly Phe Thr Ser Leu Arg Leu His Glu Ser
                   165                 170                 175
   gcg ttt ctg gtg gcg atg cct gaa gag cat cat ctc tca tca ttt tcc         816
   Ala Phe Leu Val Ala Met Pro Glu Glu His His Leu Ser Ser Phe Ser
               180                 185                 190
   acc gtc ccg ctg gaa gcg cta cgt gac gag tat ttt gtt aca atg ccg         864
   Thr Val Pro Leu Glu Ala Leu Arg Asp Glu Tyr Phe Val Thr Met Pro
           195                 200                 205
   ccc gtt tac act gac tgg gat ttt ttg cag cga gtt tgc cag cag gtg         912
   Pro Val Tyr Thr Asp Trp Asp Phe Leu Gln Arg Val Cys Gln Gln Val
       210                 215                 220
   gga ttt tca ccg gtt gtt att cgc gaa gtt aat gaa ccg caa acg gta         960
   Gly Phe Ser Pro Val Val Ile Arg Glu Val Asn Glu Pro Gln Thr Val
   225                 230                 235                 240
   ctc gct atg gtc agt atg ggc att ggt atc aca ttg ata gcg gac agc        1008
   Leu Ala Met Val Ser Met Gly Ile Gly Ile Thr Leu Ile Ala Asp Ser
                   245                 250                 255
   tac gca caa atg aac tgg cca ggt gtc att ttc cgt ccg ctc aaa cag        1056
   Tyr Ala Gln Met Asn Trp Pro Gly Val Ile Phe Arg Pro Leu Lys Gln
               260                 265                 270
   cgc atc cct gca gat tta tat att gtt tat gaa aca cag cag gtg acg        1104
   Arg Ile Pro Ala Asp Leu Tyr Ile Val Tyr Glu Thr Gln Gln Val Thr
           275                 280                 285
   ccc gcg atg gtt aag ctg ttg gct gca ttg aca cag taggtgagga             1150
   Pro Ala Met Val Lys Leu Leu Ala Ala Leu Thr Gln
       290                 295                 300
   gacaggaatt gccggaaata gtagtctgct aagtgaagcg catcaggcaa tttcgcaaat      1210
   cgcagatagc aaaaggtacc gt                                               1232
<210> 17
   <211> 300
   <212> PRT
   <213> Escherichia coli
<400> 17
   Tyr Met Glu His Leu Ile Met Glu Arg Val Tyr Arg Thr Asp Leu Lys
   1               5                   10                  15
   Leu Leu Arg Tyr Phe Leu Ala Val Ala Glu Glu Leu His Phe Gly Arg
               20                  25                  30
   Ala Ala Ala Arg Leu Asn Met Ser Gln Pro Pro Leu Ser Ile His Ile
           35                  40                  45
   Lys Glu Leu Glu Asn Gln Leu Gly Thr Gln Leu Phe Ile Arg His Ser
       50                  55                  60
   Arg Ser Val Val Leu Thr His Ala Gly Lys Ile Leu Met Glu Glu Ser
   65                  70                  75                  80
   Arg Arg Leu Leu Val Asn Ala Asn Asn Val Leu Ala Arg Ile Glu Gln
                   85                  90                  95
   Ile Gly Arg Gly Glu Ala Gly Arg Ile Glu Leu Gly Val Val Gly Thr
               100                 105                 110
   Ala Met Trp Gly Arg Met Arg Pro Val Met Arg Arg Phe Leu Arg Glu
           115                 120                 125
   Asn Pro Asn Val Asp Val Leu Phe Arg Glu Lys Met Pro Ala Met Gln
       130                 135                 140
   Met Ala Leu Leu Glu Arg Arg Glu Leu Asp Ala Gly Ile Trp Arg Met
   145                 150                 155                 160
   Ala Thr Glu Pro Pro Thr Gly Phe Thr Ser Leu Arg Leu His Glu Ser
                   165                 170                 175
   Ala Phe Leu Val Ala Met Pro Glu Glu His His Leu Ser Ser Phe Ser
               180                 185                 190
   Thr Val Pro Leu Glu Ala Leu Arg Asp Glu Tyr Phe Val Thr Met Pro
           195                 200                 205
   Pro Val Tyr Thr Asp Trp Asp Phe Leu Gln Arg Val Cys Gln Gln Val
       210                 215                 220
   Gly Phe Ser Pro Val Val Ile Arg Glu Val Asn Glu Pro Gln Thr Val
   225                 230                 235                 240
   Leu Ala Met Val Ser Met Gly Ile Gly Ile Thr Leu Ile Ala Asp Ser
                   245                 250                 255
   Tyr Ala Gln Met Asn Trp Pro Gly Val Ile Phe Arg Pro Leu Lys Gln
               260                 265                 270
   Arg Ile Pro Ala Asp Leu Tyr Ile Val Tyr Glu Thr Gln Gln Val Thr
           275                 280                 285
   Pro Ala Met Val Lys Leu Leu Ala Ala Leu Thr Gln
       290                 295                 300
<210> 18
   <211> 270
   <212> DNA
   <213> Escherichia coli
<400> 18
   ccaagcttag cataattccc tatgccgatc gttattagta gattgcttaa taaagtggtt     60
   tgctaaaaac gacatattgt taatgcaaag gaattaatat cgccaataca gtttttaccg    120
   tggattaata gagaaaagat ataaatccat cgatatcgaa tcgcctattg aatgccgctg    180
   agaaaagtgt attggtaaga gccgggacaa cctcgctata aaaaggcgtt agattccacc    240
   ctacagaaaa aggataccca tggtgaccac                                     270
<210> 19
   <211> 272
   <212> DNA
   <213> Escherichia coli
<400> 19
   ccaagcttag cataattccc tatgccgatc gttattagta gattgcttaa taaagtggtt     60
   tgctaaaaac gacatattgt taatgcaaag gaattaatat cgccaataca gtttttaccg    120
   tggattaata gagaaaagat ataaatccat cgatatcgaa tcgcctattg aatgccgctg    180
   agaaaagtgt attggtaaga gccgggacaa cctcgctata aaaaggcgtt agattccacc    240
   ctacagaaaa aggatacata tgtctcaggt tc                                  272
<210> 20
   <211> 270
   <212> DNA
   <213> Escherichia coli
<400> 20
   agcataattc cctatgccga tcgttattag tagattgctt aataaagtgg tttgctaaaa     60
   acgacatatt gttaatgcaa aggaattaat atcgccaata cagtttttac cgtggattaa    120
   tagagaaaag atataaatcc atcgatatcg aatcgcctat tgaatgccgc tgagaaaagt    180
   gtattggtaa gagccgggac aacctcgcta taaaaaggcg ttagattcca ccctacagaa    240
   aaaggatatg tatgtctcag gttcaatttt                                     270

## Description

The present invention relates to novel vectors and nucleic acid sequences, which comprise promoters which are induced by nucleotides (with or without a phosphate group). The invention further relates to expression vectors utilising elements of the xanthosine operon and methods for the production of heterologous proteins.

Expression of cloned genes introduced into bacteria is still the most widely used mechanism for producing large amounts of a protein of interest for diagnostic and therapeutic purposes. In order to efficiently produce proteins in a prokaryotic host, a strong, regulated promoter is an essential element of the expression system. Promoters and expression systems already known in the art include the bacteriophage lambda pL and pR promoters, which can be regulated by a temperature-sensitive repressor which represses transcription from that promoter at low temperatures and allows expression of the heterologous protein by means of temperature induction (EP-A-41767), or alternatively by use of an inducible antirepressor system which can be induced using isopropyl-β-D-thiogalactopyranoside (IPTG) at lower temperatures, whilst still employing the strong pL/pR promoters (WO 98/48025). However, high temperatures routinely lead to the formation of the heterologous protein as insoluble aggregates, or inclusion bodies, which subsequently require costly refolding steps using toxic chemicals.

Other promoters include the lac operon and derivatives, such as the trp-lac promoter or tac promoter, (EP-A-67540) which has been used to produce high levels of proteins in *E. coli.* This promoter is induced in the presence of IPTG. In order to subject the promoter to repression it must be used together with a plasmid overproducing the lac repressor, or in an E. *coli* strain which produces lac repressor protein. T7 promoter-based systems (US5869320) utilise the T7 RNA polymerase, inducibly expressed, are routinely used in research laboratories for the overproduction of proteins, again using IPTG. However, the strength of such promoters again can lead to the production of inclusion bodies, or aggregates, of the heterologous proteins.

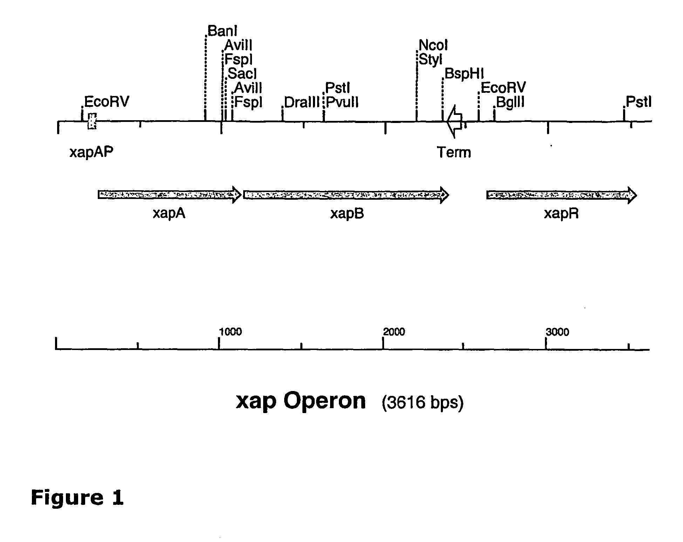

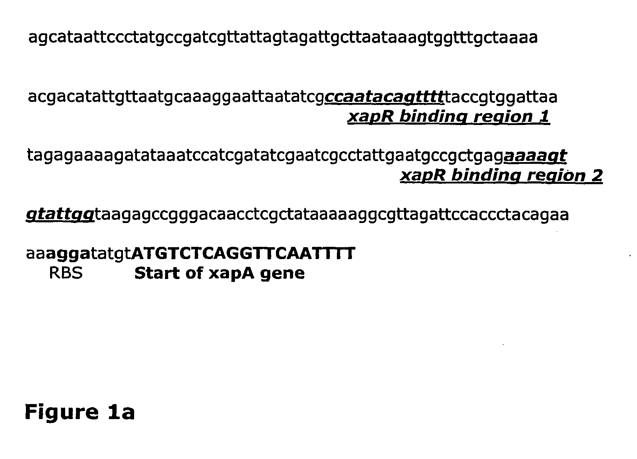

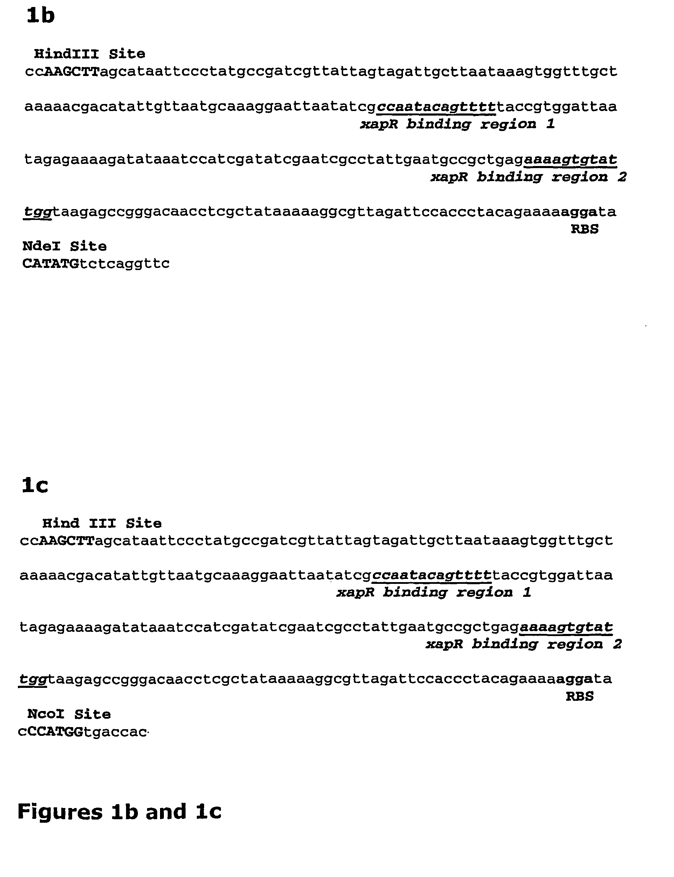

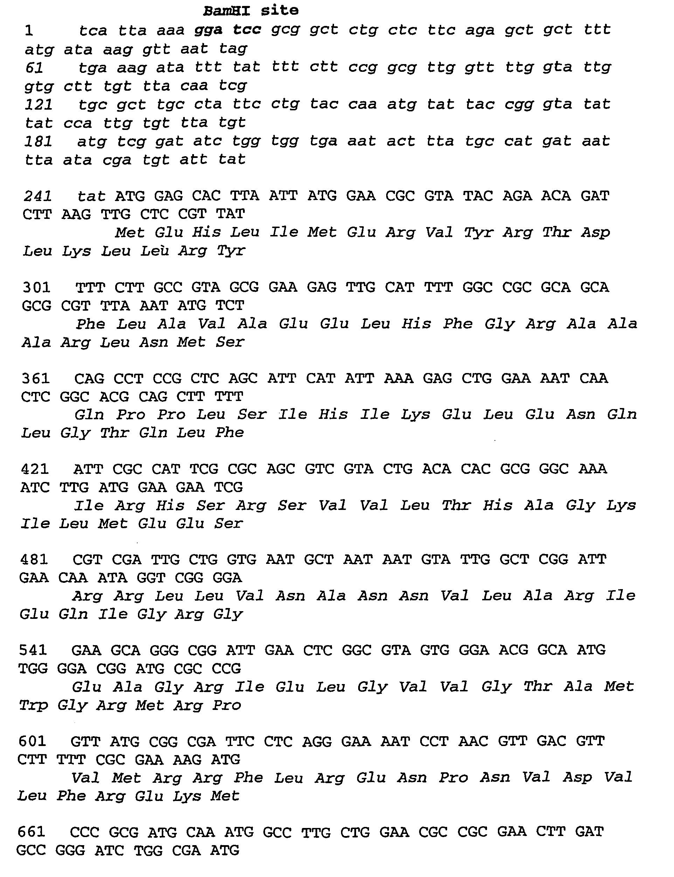

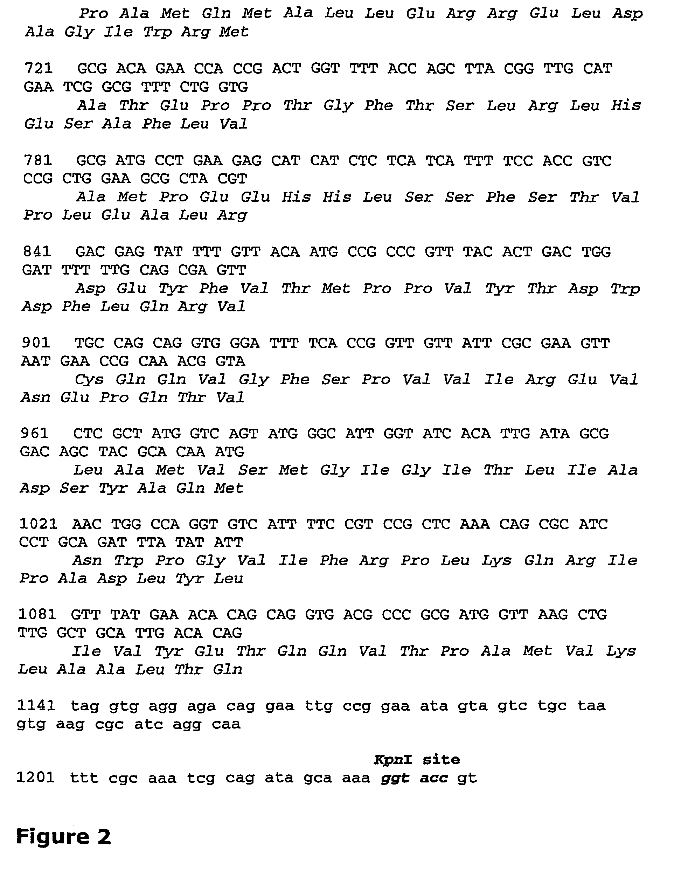

*Escherichia coli,* a Gram-negative bacterium, is able to grow on xanthosine as the sole carbon source (Hammer-Jespersen *et al*., 1980). This occurs via the induction and utilisation of genes in the xanthosine operon, comprising of at least three genes, located at approximately 52' on the *E. coli* chromosome (Figure 1) (Seeger *et al*., 1995). The genes in the operon are xapA, xapB and xapR. XapA encodes for xanthosine phosphorylase, which catalyses the breakdown of the N-glycosidic bond in nucleoside molecules, resulting in a free base and a pentose-1-phosphate. The free bases can then be subsequently used in the purine and pyrimidine salvage pathways and also as a nitrogen source (Nygaard, 1983), and the pentose molecule can subsequently be utilised as a carbon source, hence the ability of *E. coli* to grow on xanthosine minimal media. The function of the xapB protein has not been fully elucidated, but would appear by homology to other proteins and by its cellular location to be a membrane transport protein, almost certainly involved in transporting nucleosides across the cell membrane (Seeger, *supra*). XapR shows significant homology to the LysR family of transcriptional regulatory proteins, DNA-binding proteins involved in gene activation (Henikoff *et al*., 1988), and appears to be constitutively expressed. The xapA and B genes appear to be co-expressed, and xapA expression is found only in the presence of xanthosine, and the xapR protein (Seeger, *supra*). The xapR protein, if a true LysR family member, would bind to specific regions within the xapA promoter, enabling the subsequent binding of E. *coli* RNA Polymerase, and subsequent transcription of the xapA (and xapB) genes. Such DNA-binding proteins appear to recognise an inverted complimentary consensus sequence ATATTGTTT (Bohannon 1989), and there appears to be such a region within the xapA promoter region (Figure 2), at 120bp upstream of the transcription start (CCAATACAGTTTT), and the corresponding sequence at 40bp upstream, overlapping the -35 region (AAAACTGTATTGG) (Seeger, *supra*). Sequences of the genes comprising the xanthosine operon of *E. coli* have been deposited at several online public databases, e.g. the GenBank database, accessible through the home page of the National Center for Biotechnology Information (NCBI), Bethesda, Maryland, USA at www.ncbi.nlm.nih.gov, using the accession numbers AE000328, D90869, D90870 or X73828.

The current invention relates to the expression of heterologous proteins using a novel expression vector and system, which avoids the problems of insoluble inclusion bodies associated with temperature induction or very strong promoters, and which does not require the use of toxic inducers, such as IPTG, which would require costly validation procedures to verify its absence when producing therapeutic proteins.

Accordingly, a first aspect of the present invention provides a vector, comprising a promoter which can be operable linked to a gene encoding a heterologous protein, wherein the promoter induces expression of any operably linked heterologous protein, in the presence of nucleotides, which nucleotides may or may not have a phosphate group and wherein the promoter sequence, is a XapA promoter which comprises a ribosomal binding site having the following AGGAGGxxxxx or AGGAGGxxxxxx or AGGAGAxxxxx or AGGAGAxxxxxx.

The phosphate group may result in the molecule being a phosphate ester. There may be one or more phosphate groups attached.

The vector may be any, including a plasmid or a bacteriophage. Preferably, the vector is an inducible expression vector.

The induction of the promoter is by the presence of nucleotides in the media (usually culture media).

The term "nucleotides with or without a phosphate group" include both nucleotides and nucleosides.

In accordance with the present invention, nucleotides include any one or more nucleoside molecule containing one or more phosphate groups covalently linked to the sugar molecule, which can be of the oxy, deoxy or dideoxy form. Examples of such include: adenosine triphosphate (ATP), guanosine triphsophate (GTP), cytidine triphsophate (CTP), thymidine triphsophate (TTP), and uridine triphosphate (UTP). The deoxy forms of these (commonly written as dATP, dCTP, dGTP and dTTP and commonly known as bases) form the basic structure of DNA. The oxy forms of these (commonly written as ATP or rATP, CTP or rCTP, GTP or rGTP, UTP or rUTP and commonly known as RNA bases) form the basic structure of RNA.

In accordance with the present invention, nucleosides include any one or more compound comprising a purine or pyrimidine joined by an N-glycosidic link to a sugar, particularly an oxy- or deoxy- or dideoxy-ribose sugar molecule. Examples of such nucleosides include the oxy, deoxy and dideoxy- forms of adenosine, guanosine, inosine, cytidine, thymidine, uridine, xanthosine, and derivatives thereof.

Xanthosine is known under the USPTO classification is: 536/27.8. It is also known as xanthosine, or 9-beta-D-ribofuranosyl xanthine (Chemical Abstract Service number 5968-90-1), and includes the compound either as an anhydrous salt or a dihyrate salt. Synonymous names for xanthosine are: Xanthine riboside, 9-beta-D-ribofuranosyl-9H-purine-2,6-diol and 9-beta-D-ribofuranosyl-9H-purine-2,6-(1H,3H)-dione.

Preferred concentrations of nucleotides as an inducer are in the range of from 0.01 to 10 mg/ml, more preferably from 0.1 to 1 mg/ml.

Any promoter can be used which can be operably linked to a gene encoding a heterologous protein and wherein the promoter induces expression of any such operably linked heterologous protein, in the presence of nucleotides.

Steps to operably link nucleic acid sequences are well known in the art and are currently predominantly based on the use of restriction enzymes to cut nucleic and polymerases to join nucleic acid. Examples of such steps are shown in the example section.

The promoter may be directly or indirectly induced by the presence of nucleotides. Suitable promoters include those from a xapA gene. Any xapA gene is acceptable. The xapA promoter region from *E. coli* is set out in Figure 1a. Modified xapA promoter regions, included within the present invention, are shown in Figures 1b and 1c. These modified regions have useful restriction enzymes site included.

In addition to this sequence, any modified sequence can be used which sequence induces expression of an operably linked nucleic acid sequence in the presence of nucleotides or nucleosides. For example, the modified sequence may be a substantially homologous sequence. A substantially homologous sequence preferably has at least 75, 76, 77, 78, 79, 80, 81, 82, 83, 84, 85, 86, 87, 88, 89, 90, 91, 92, 93, 94, 95, 96, 97, 98 or 100% sequence identity with the defined sequence.

"% identity" is a measure of the relationship between two nucleic acid or polypeptide sequences, as determined by comparing their sequences. In general, the two sequences to be compared are aligned to give a maximum correlation between the sequences. The alignment of the two sequences is examined and the number of positions giving an exact amino acid or nucleotide correspondence is determined, and divided by the total length of the alignment, and the result is multiplied by 100 to give a % identity. The % identity may be determined over the whole length of the sequence to be compared, which is particularly suitable for sequences of the same or similar lengths or for sequences which are highly homologous, or over shorter defined lengths which is more suitable for sequences of unequal lengths and with a lower homology.

Methods for comparing the identity of two or more sequences are known in the art. For example, programs available in the Wisconsin Sequence Analysis Package version 9.1 (Devereux J et al., Nucl Acid Res 12 387-395 (1984), available from Genetics Computer Group, Madison, Wisconsin, USA), such as BESTFIT and GAP may be used.

BESTFIT uses the "local homology" algorithm of Smith and Waterman (Advances in Applied Mathematics, 2:482-489, 1981) and finds the best single region of similarity between two sequences. BESTFIT is more suited to comparing two polynucleotide or two polypeptide sequences which are dissimilar in length, the program assuming that the shorter sequence represents a portion of the longer. In comparison, GAP aligns two sequences finding a "maximum similarity" according to the algorithm of Neddleman and Wunsch (J. Mol. Biol. 48:443-354,1970).

GAP is more suited to comparing sequences which are approximately the same length and an alignment is expected over the entire length. Preferably, the parameters "Gap Weight" and "Length Weight" used in each program are 50 and 3 for polynucleotide sequences and 12 and 4 for polypeptide sequences, respectively. Preferably, % identities and similarities are determined when the two sequences being compared are optimally aligned.

Other programs for determining identity and/or similarity between sequences are also known in the art, for instance the BLAST family of programs (Altschul et al., J. Mol. Biol., 215:403-410, (1990) and Altschul et al., Nuc Acids Res., 25:289-3402 (1997), available from the National Center for Biotechnology Information (NCBI), Bethesda, Maryland, USA and accessible through the home page of the NCBI at www.ncbi.nlm.nih.gov) and FASTA (Pearson W.R. and Lipman D.J., Proc. Nat. Acac. Sci., USA, 85:2444-2448 (1988), available as part of the Wisconsin Sequence Analysis Package).

In relation to the present invention, "stringent conditions" refers to the washing conditions used in a hybridisation protocol. In general, the washing conditions should be a combination of temperature and salt concentration so that the denaturation temperature is approximately 5 to 20°C below the calculated Tₘ of the nucleic acid under study. The Tₘ of a nucleic acid probe of 20 bases or less is calculated under standard conditions (1M NaCl) as [4°C x (G+C) + 2°C x (A+T)], according to Wallace rules for short oligonucleotides. For longer DNA fragments, the nearest neighbour method, which combines solid thermodynamics and experimental data may be used, according to the principles set out in Breslauer et al., PNAS 83: 3746-3750 (1986). The optimum salt and temperature conditions for hybridisation may be readily determined in preliminary experiments in which DNA samples immobilised on filters are hybridised to the probe of interest and then washed under conditions of different stringencies. While the conditions for PCR may differ from the standard conditions, the Tₘ may be used as a guide for the expected relative stability of the primers. For short primers of approximately 14 nucleotides, low annealing temperatures of around 44°C to 50°C are used. The temperature may be higher depending upon the base composition of the primer sequence used. Suitably stringent conditions are those under which non-specific hybridisation are avoided. Suitable stringent conditions are 0.5xSSC/1%SDS/58°C/30mins for a 21mer oligonucleotide probe.

The vector may further comprise a regulatory element which the nucleotides interact with in order to regulate promoter. The regulatory element is preferably a nucleic acid sequence. Where the regulatory element is a xapR gene, it may need to be expressed in order for the expressed protein to interact with the nucleotides and regulate the promoter, for increased expression of any heterologous protein.

Most preferably, in accordance with the invention, the regulatory element is from a xapR gene. Any xapR gene is acceptable. The xapR gene from *E*. *coli* is set out in Figure 2.

In addition to this given xapR sequence, any modified xapR sequence can be used which acts in the same manner on the promoter. For example, the modified sequence may be a substantially homologous xapR sequence (as defined above). The xapR sequence may need to be expressed. Suitable modified xapR sequences are described in Jorgensen and Dandanell, 1999.

The expressed xapR protein is believed to bind to specific regions within the xapA promoter, enabling the subsequent binding of RNA Polymerase, and subsequent transcription of any operably linked heterologous gene.

In order for the xapR, or other regulatory element to be expressed, it may be necessary for the vector to comprise a further promoter which is operably linked to the regulatory element to control its expression. Preferably, such a promoter is a constitutive or inducible promoter, such that the regulatory element is constitutively expressed. The further promoter may be a xapR promoter or any other know constitutive or inducible promoter, such as the lac, trc, lambda pR, lambda pL or trp promoters.

In an alternative, the regulatory element, which regulates expression by the promoter may be present but not as part of the vector on which the inducible promoter is present. The regulatory element (preferably with constitutive or inducible promoter) may be present in a cell as part of another vector, or integrated into the cell's genome.

The vector of the first aspect of the invention may be with or without an operably linked gene encoding a heterologous protein. The form of the vector without such a gene can be termed an "empty cassette", which enables the addition of any such gene, for use in expression of that gene. The addition is by steps known in the art (predominantly the use of cutting restriction enzymes and joining polymerases), examples of which are given in the examples.

Any gene encoding any heterologous protein can be inserted into the vector of the first aspect of the invention. Examples of such genes include those which encode cytokines, hormones, chemokines, enzymes, antigens etc (preferably human forms). Cytokines include interleukins, e.g. human interleukin-4. Hormones include human growth hormone. The heterologous protein produced can be a fusion protein, e.g. attached to a leader peptide, for secretion into the periplasmic space or extracellular media, using such leader peptides as ompA or pelB, or a secondary polypeptide, operably linked to the heterologous protein, the function of which can be to prevent proteolytic degradation of the heterologous protein, or to provide an affinity tag for purification, or to assist in solubilisation of the heterologous protein. Examples of such secondary polypeptides include thioredoxin, maltose binding protein, histidine tags, and such secondary polypeptides may or may not include cleavage sites, for removal of the secondary polypeptide by selective cleavage using chemical or enzymatic means. Examples of such include cyanogens bromide, trypsin, enterokinase and Factor Xa.

A second aspect of the invention provides an expression vector, comprising an isolated nucleic acid, comprising a regulatory gene from xapR of the xanthosine operon together with a promoter from a xapA gene.

The xapA promoter may be as herein described in Figure la and includes any substantially homologous sequence, also as hereinbefore described.

The xapR gene may be as herein described in Figure 2 and includes any substantially homologous sequence, also as hereinbefore described.

The nucleic acid may also comprise a further promoter which is operably linked to the xapR gene. Such a promoter is as described for the first aspect of the invention. It may be from the xapR promoter or may be any inducible or constitutive promoter. The xapR promoter may be as herein described in Figure 2.

The vector or expression vector of the first or second aspect may, of course, comprise other elements, such as a phenotypic selection marker, such as antibiotic resistance, replication gene(s), etc.

A third aspect of the invention provides a xapA promoter sequence which comprises a ribosomal binding site having any one of the following sequences:
AGGAGG xxxxx
AGGAGG xxxxxx
AGGAGA xxxxx
AGGAGA xxxxxx
wherein x is any base.

The optional bases (x) may be those as described in Example 2 (taccc or tatccc).

The nucleic acid sequences of the third aspect may comprise the 5' sequences, also as shown in Example 2. The nucleic acid sequences of the third aspect may be part of a promoter sequence. The promoter sequence may be from a xapA promoter. The nucleic acid sequences of the third aspect of the inventor may be part of a vector, as described according to the first aspect of the invention.

A fourth aspect of the invention provides host cell, which comprises one or more vectors or nucleic acid sequences, according to any one of the first, second or third aspects of the invention. Such a host cell may be any, preferably those which can be used in culture to provide protein production on a commercial scale. Examples of such host cells include *E. coli, Bacillus sp.,* and yeasts such as *Saccharomyces* and *Pichia.*

The vector and/or nucleic acid is introduced to the host cell by any technique, such as transformation, electroporation etc.

A fifth aspect of the invention provides a method for the expression of a heterologous protein, the method comprising culture of host cells, according to the fourth aspect of the invention under conditions which induce the expression of the heterologous protein. Such methods are well known in the art (see for example "Manual of Industrial Microbiology and Biotechnology" "2nd Edition. Demain A, & Davies J, 1999). Suitable conditions include culturing said transformed host cells in a culture medium containing nutrients that meet the requirements of said host cells, such as carbon and nitrogen sources, vitamins and trace elements, together with a compound suitable for selecting those cells containing the expression vector, which may contain a selective marker, such as an antibiotic resistance gene. Cells are cultivated under conditions to achieve optimal growth, with regard to pH, typically ranging from pH6-8, temperature, which typically ranges from 20-42C, and also with a provision of oxygen, ranging from 10-50%, with 30% being a typical optimal value. Cells are grown under such conditions until a suitable density is reached, at which point the inducer is added in sufficient quantity to achieve maximal induction of the expression vector within the cells, and consequently allowing the expression of the protein of interest. Induction is allowed to continue for the optimal, empirically-defined time, at which point the protein of interest can then be harvested.

The method may further comprise purification of the heterologous protein. Such purification steps are also known in the art (see for example "Protein Purification", 2nd Edition, Janson, J-C & Ryden, L, 1998). Basic steps for the extraction of intracellularly produced heterologous protein would typically involve a step of breaking open the cells, by a variety of means, e.g. homogenisation, or chemical lysis, or freeze-thawing, or ultrasonication. Intracellular protein which was present in the soluble extract could then be captured by a wide variety of chromatographic steps well known in the art, e.g. ion-exchange, hydrophobic interaction, affinity chromatography, reverse-phase chromatography, size exclusion or gel filtration and ultrafiltration. Typically, additional chromatographic steps are employed to further purify the heterologous protein away from contaminating host cell proteins and impurities, resulting in a highly purified protein. Intracellular protein which was present in the insoluble part of the extract, e.g. present as inclusion bodies, could be solubilised using a number of methods known in the art, using such compounds as guanidine, urea or sodium dodecyl sulphate for example, and subsequently purified using one or more chromatographic steps as mentioned above. Heterologous protein which was produced in the periplasmic space, using an expression vector which employed a leader peptide as described above, could be released from said space by employing an osmotic shock, to release the contents of the periplasmic space into the media, using such compounds as sucrose, or magnesium sulphate, or lysozyme/EDTA, following which, one or more chromatographic steps are employed to purify said protein.

A seventh aspect of the invention provides the use of one or more vectors or nucleic acid sequences, according to any one of the, second or third aspects of the invention in the production of a heterologous protein.

All preferred features of the various aspects of the invention apply to each other, *mutatis mutandis.*

It is the object of this invention to provide expression vectors, comprising a promoter and regulatory repressor, which is derived from an operon for xanthosine metabolism in *E. coli*, for the expression of heterologous proteins of commercial value. In one particular embodiment of the invention, the xapR regulatory protein, together with its promoter, are isolated and introduced into an expression vector, together with a heterologous gene operably linked to the promoter from the xapA gene. The resulting expression vector is transformed into a suitable host cell, which is subsequently grown under suitable conditions to achieve optimal growth. Expression of the heterologous protein does not occur until the addition of xanthosine, which subsequently activates the xapR protein, enabling it to bind to the xapA promoter region, allowing expression of the heterologous protein from the xapA promoter.

It is a further object of this invention to provide novel ribosmal binding site sequences for the xanthosine operon, which have increased levels of expression of heterologous proteins, whereby specific changes have been made to the natural ribosmal binding site of the xapA operon, as detailed in the first embodiment, and whereby a screening method has been utilised to identify those mutations resulting in increased expression levels

It is a further object of this invention to provide a method for production of heterologous proteins using an expression system inducible with nucleotides, such as xanthosine. In the examples below, the construction of the xanthosine-inducible expression vectors is described, and the utility of the invention is illustrated using the production of human growth hormone (hGH) and human interleukin-4, in *E. coli.*

It is apparent to a person skilled in the art that other genes can be expressed in the system described here. In addition, such a system could be integrated into the genome of *E. coli,* or other organisms, or a similar expression system could be constructed utilising homologous genes from xanthosine operons from other organisms, including such genera as Salmonella, Pseudomonas, Bacillus or Streptococcus, etc.

The present invention is described with reference to the accompanying figures, in which:
Figure 1a shows the nucleotide sequence of the *E. coli* xapA promoter region.
   (RBS - Ribosomal Binding Site)
Figure 1b shows the nucleotide sequence of the *E. coli* xapA promoter region, together with new *Hind*III and *Nde*I restriction sites
Figure 1c shows the nucleotide sequence of the *E. coli* xapA promoter region, together with new *Hind*III and *NcoI* restriction sites
Figure 2 shows the nucleotide and amino acid sequence of the *E. coli* xapR gene (upper case) and the xapR promoter region (lower case italics), together with new restriction sites *Bam*HI and *Kpn*I.
Figure 3 shows the vectors pUC19x, pXap1a and pXap1b
Figure 4 shows the map of pXap1b-βgal
Figure 5 shows the maps of Xap 1a- hGH and of pXap-IL4
Figure 6 shows the protein analysis of pXap-hGH fractions:
   (A)-SDS-PAGE; (B)-Western-blot.
   Lanes:
   1) hGH protein standard; 2) Molecular Weight Marker;
   Lanes 3-7 LB Medium
   3) Oh induction; 4) 2h induction; 5) 3h induction; 6) 4h induction; 7) 6h induction
   Lanes 8-12 Defined medium
   8) Oh induction; 9) 2h induction; 10) 3h induction; 11) 4h induction;
   12) 6h induction.
   In part A of the figure, the protein of interest is the one most clearly and abundantly present (indicated by the arrow) and which has migrated with the protein standard in lane 1.
   In part B, the Western Blot shows that the protein sought (by means of a specific antibody) is present.

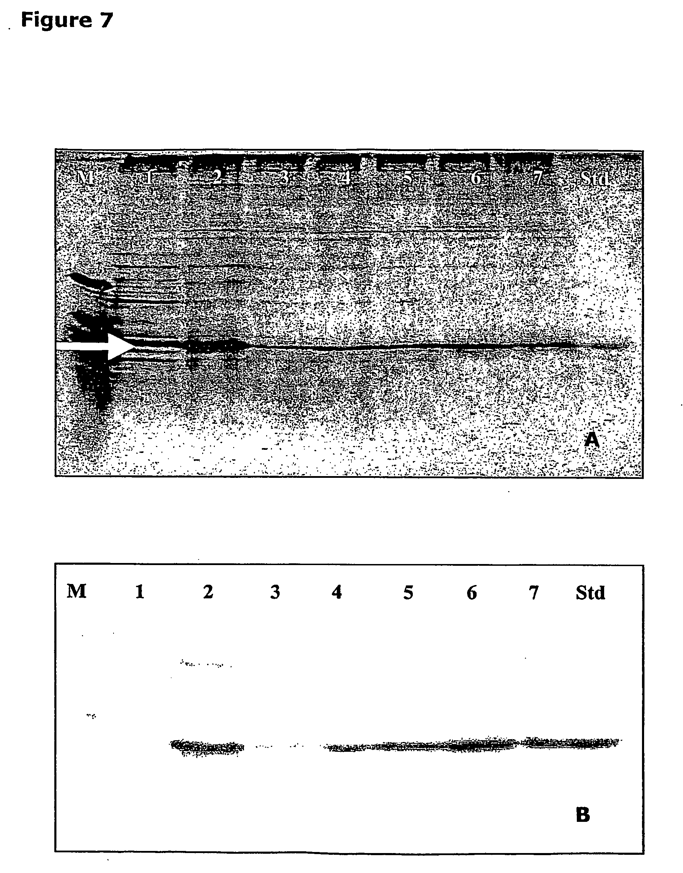

Figure 7 shows the protein analysis of pXap-IL4 fractions at 20°C and 28°C:
   (A) SDS-PAGE; (B) Western Blot.
   Lane M: Protein Markers.
   Lanes 1-4: 20C, 0h, 2 h, 5 h and overnight induction.
   Lanes 5-7: 28C, after 2 h, 5 h and overnight induction. Lane Std: IL-4 standard.
   In part A of the figure, the protein of interest is the one most clearly and abundantly present (indicated by an arrow) and which has migrated with the protein standard in the lane marked Std on the right hand side..
   In part B, the Western Blot shows that the protein sought (by means of a specific antibody) is present.

The present invention is now described with reference to the following non-limiting examples:

### EXAMPLES

The materials and methods used are described below, and the invention is illustrated in the examples. In support of most of the methods, reference is made to the following books: Sambrook et al., (1989) "Molecular Cloning: A Laboratory Manual", 2nd Edition, and Miller, J. (1972) "Experiments in Molecular Genetics", both from Cold Spring Harbor Press, Cold Spring Harbor, NY, USA, and Dieffenbach C.W & Dveksler G.S. (1995); PCR Primer: A Laboratory Manual. Cold Spring Harbor Laboratory, Cold Spring Harbor, New York.

### Example 1: Xanthosine-Inducible Expression Vector construction

### PCR amplification of the XapR gene (regulatory protein).

The XapR gene, together with its own promoter sequence, was amplified from *E. coli* genomic DNA from strain MG1655 (ATCC # 700926), using forward primer xap2 (SEQ_ID1) 5'-ACGGTACCTTTTGCTATCTGCGATTTGCG-3' and reverse primer xap5 (SEQ_ID2) 5'-CTCATTAAAAGGATCCGCGGCTCTGCTCTTCAG-3'. The reaction mixture contained *Pfu* Buffer (20mM Tris-HCl pH8.8 at 25°C; 10mM ammonium sulphate; 10mM potassium chloride; 0.1% Triton X-100; 0.1 mg/ml bovine serum albumin and 2mM magnesium sulphate), 0.25mM of each dATP, dCTP, dGTP and dTTP, 500ng of *E*. *coli* genomic DNA, 50pmol of each primer, 1.25 units of *Pfu* DNA polymerase (MBI Fermentas) and water to a final volume of 50ul. The PCR amplification method used was as follows:

| 1 cycle of: | | |
|---|---|---|
| | 94°C | 5 minutes initial denaturation |
| | 72°C | hold (polymerase addition) |

| 14 cycles of: | | |
|---|---|---|
| | 65°C | 1 minute annealing (-1°C decrease per cycle) |
| | 72°C | 5 minutes elongation |
| | 94°C | 1 minute denaturation |

| 19 cycles of: | | |
|---|---|---|
| | 55°C | 1 minute annealing |
| | 72°C | 5 minutes elongation (+0.02 minutes increment per cycle) |
| | 94°C | 1 minute denaturation |
| | 55°C | 1 minute annealing |
| | 72°C | 7 minutes final elongation |

A 1.2 kb fragment corresponding to the xapR gene was amplified. The DNA fragment resulting from this PCR reaction was analysed on a 0.8% TAE/agarose gel stained with 0.1ug/ml ethidium bromide. The fragment was subsequently purified from the gel.

### PCR Amplification of the xapA promoter.

The xapA promoter region was PCR amplified from *E. coli* genomic DNA from strain MG1655 using forward primer xap1 (SEQ_ID3)
5'-CCAAGCTTAGCATAATTCCCTATGCCGATC-3', and phosphorylated reverse primer xap4 (SEQ_ID4) 5'-GAACCTGAGACATATGTATCCTTTTG-3', or phosphorylated xapNco R (SEQ_ID5)
5'-GTGGTCACCATGGGTATCCTTTTTCTG TAG G-3', using the reaction mixture as described above. The amplification method used was as follows:

| 1 cycle of: | | |
|---|---|---|
| | 94°C | 5 minutes initial denaturation |
| | 72°C | Hold (polymerase addition) |

| 35 cycles of: | | |
|---|---|---|
| | 65°C | 1 minute annealing |
| | 72°C | 0.5 minute elongation |
| | 94°C | 1 minute denaturation |

| 1 cycle of: | | |
|---|---|---|
| | 65°C | 1 minute annealing |
| | 72°C | 3 minutes final elongation |

A 270 bp fragment corresponding to the xapA promoter was amplified. The DNA fragment resulting from these PCR reactions was analysed on a 1% TAE/agarose gel stained with 0.1ug/ml ethidium bromide, and again purified from the gel.

### Expression Vector construction

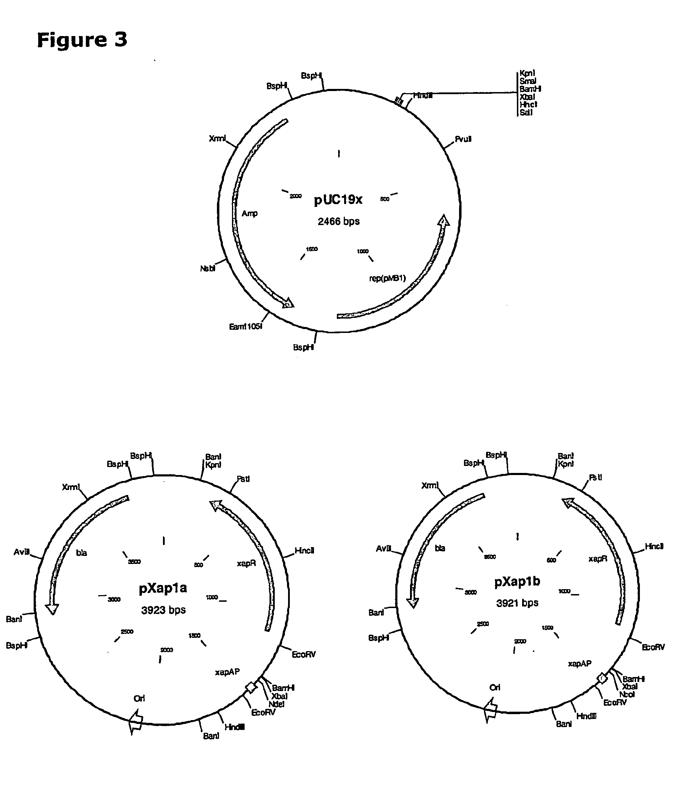

The first vector to be constructed was pUC19X. To construct this vector, pUC19 was digested with *Sac*I and *Nde*I and the resulting overhangs were filled-in using T4 DNA polymerase (Promega), according to the manufacturer's instructions. The resulting 2.4 kb fragment was then self-ligated and transformed into XL1-Blue competent cells (Stratagene). The purified xapR gene and the xapA promoter PCR products, as described above, were inserted into pUC19X, using restriction endonucleases *Bam*HI and *Kpn*I from the xapR gene and *Hind*III and *Hinc*II for the xapA promoter. The resulting plasmids were called pXap1a, and pXap1b, differing only by the presence of either a unique *Nco*I site or *Nde*I site. Maps of the initial construct and the expression vector constructs are given in Figure 3.

The construct is designed such that the xapR protein, under the control of its own promoter (incorporated into the PCR product as described above), is expressed constitutively. The xapA promoter is inserted into a suitable region of the vector, and allows for the cloning of heterologous genes into the NcoI site (pXap1a) or NdeI site (pXap1b). This ensures that any such heterologous gene is correctly positioned with respect to the promoter region of the xapA promoter, with respect to the transcriptional start site, the ribosomal binding site and the RNA Polymerase binding site of the promoter, allowing correct expression of the heterologous protein, upon induction with xanthosine, but not allowing non-induced expression in the absence of xanthosine.

### Example 2. Creation of ribosomal binding site mutants and activity analysis.

### Expression analysis of pXap1b using β-galactosidase

The E. *coli* β-galactosidase (β-Gal) gene was initially obtained by PCR amplification of the β-gal gene from E. *coli* genomic DNA from strain MG1655, using the amplification methods described above, with the following primers - β-Gal-1 (SEQ_ID6) 5'- ATATGGGCCCATGGATCCCGTCGTTTTAC-3', and β-Gal-2 (SEQ_ID7) 5'- AGTGTGAAGCTTATTATTTTTGACACCAG-3'. The PCR fragment was then ligated into the expression vector pSE420 (Invitrogen), using NcoI/HindIII double digests, and used to transform chemically competent DH5 cells (Gibco-BRL). Positive clones were identified by restriction analysis.

Subsequently, the β-galactosidase gene was cloned into one of the pXap vectors, namely pXap1b, by digestion of pSE420-β-Gal with *Hind*III, followed by blunt-ending of the fragment using Klenow enzyme, and subsequent digestion with *Nco*I.

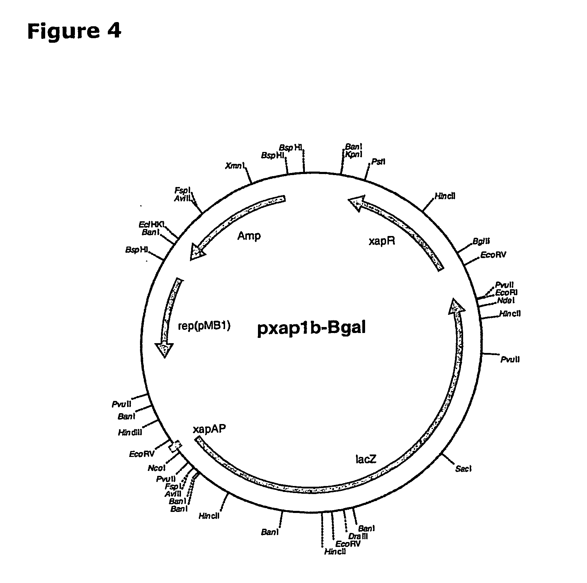

The purified fragment was then introduced into similarly digested pXap1b, so as to be in-frame with the xapA promoter. After ligation and transformation, the positive clones were identified by blue colour development when plated on LAXX plates (LB/Ampicillin Agar plates supplemented with 1mg/ml Xanthosine, 80ug/ml X-gal). The xanthosine induces the expression of the β-gal gene, which subsequently forms a blue coloured product on hydrolysis of the substrate X-gal. Positive blue colonies were screened for β-Gal activity by the assay of Miller (1972), and one such colony was designated plasmid pXap1b-β-Gal (Figure 4).

### Mutagenesis of the ribosomal binding site region of pXap1b-β-Gal

In order to develop novel vectors with increased expression, mutagenic PCR was performed upon plasmid pXap1b-β-Gal, in order to change the natural ribosomal binding site (RBS) and analyse for increased expression. Two such mutants were constructed, RBS1 and RBS2. RBS1 was designed to replace the natural RBS of xapA, AGGA, with the stronger AGGAGG consensus RBS sequence. RBS2 was designed to introduce a similarly strong AGGAGA RBS sequence, plus an additional base between the RBS and the start codon, as it has been observed that altering the spacing between the RBS and start codon can affect the expression levels, either positively or negatively, in other expression systems (Marquis *et al*., 1986; Chen *et al*., 1994).

Plasmid pXap1b-β-Gal was PCR amplified using the following primers:
pXapRBS1A
   5'-CCTACAGAAAAAGGAGGTACCCATGGATCCCGTCG-3' (SEQ_ID12) together with
pXapRBS1B
   5'-CGGGATCCATGGGTACCTCCTTTTTCTGTAGGGTGG-3',(SEQ_ID13), for the RBS1 mutant, &
pXapRBS2A 5'-CCCTACAGAAAAAGGAGATATCCCATGGATCCCGTCGTTTTACAACG-3' (SEQ_ID14) together with
pXapRBS2B
   5'- CGACGGGATCCATGGGATATCTCCTTTTTCTGTAGGGTGGAATCTAACG -3', (SEQ_ID15), for the RBS2 mutant, in separate reactions, using the following conditions.

The reaction mixture was as described in example 1, except that 1ng of purified pXap1b-β-Gal was used as the template. The reaction conditions used were:

| 1 cycle of: | | |
|---|---|---|
| | 94°C | 5 minutes initial denaturation |
| | 72°C | hold (polymerase addition) |

| 18 cycles of: | | |
|---|---|---|
| | 55°C | 1 minute annealing |
| | 72°C | 15 minutes elongation |
| | 94°C | 1 minute denaturation |

*Dpn*I restriction endonuclease (Fermentas) was then added to the reaction mixture and incubated at 37C for 2 hours. 5ul of the reaction mixture was used to transform chemically competent DH5 *E. coli* cells, and the transformed cells were plated out on LAXX plates as described above. Several positive blue colonies were screened and identified by restriction digestion

### Screening of the expression activity of PXap1b-β-Gal and RBS mutants.

Cultures of pXap1b-β-Gal and of positive RBS mutants, transformed into DH5 competent cells, were grown at 37°C in defined medium supplemented with ampicillin and induced with 1 mg/ml xanthosine (final concentration) in order to determine their β-galactosidase activity, as measured by the ONPG assay (Miller, 1972) (Table 1).

**Table 1 Comparison of the β-galactosidase activity of pXap1b-β-Gal and RBS mutants pXap1b-β-Gal-RBS1 and pXaplb-β-Gal-RBS2.**

| **Plasmid/Strain** | **β-Galactosidase Activity, in Miller Units** |
|---|---|
| pXap1b-β-Gal / DH5 | 16,900 |
| pXap1b-β-Gal-RBS1 / DH5 | 21,500 |
| pXap1b-β-Gal-RBS2 / DH5 | 24,600 |

It is thought (Chen *et al*., 1994) that a 5 nucleotide (nt) spacing between the RBS and the ATG start codon is the optimal spacing in *E*. *coli* promoters. This is the case in the naturally occurring xapA RBS. Altering the RBS of pXap1b-β-Gal to the consensus sequence AGGAGG resulted in higher levels of expression (RBS1), and surprisingly, increasing the spacing between the consensus sequence and the start codon (RBS2) increased expression levels further still.

RBS regions of the RBS1 and RBS2 plasmids, compared to plasmid pXap1b-β-Gal.
CCACCCTACAGAAAA**AGGA**taccc**ATG**GATC - pXap1b-β-Gal (5nt spacing)
CCACCCTACAGAAAA**AGGAGG**taccc**ATG**GATC - pXap1b-β-Gal-RBS1 (5nt spacing)
CCACCCTACAGAAAA**AGGAGA**tatccc**ATG**GATC - pXap1b-β-Gal-RBS2
(6nt spacing)

### Example 3: Expression of heterologous genes

### Expression of human growth hormone

The human growth hormone gene (hGH) was PCR amplified from plasmid phgh107 (ATCC#40011) using primers hGH-Nde
(SEQ_ID8) 5' AAGAATCCCATATGTTCCCAACCATTCCCTTATCC 3' and hGH-Rev (SEQ_ID9) 5' CGCGGATCCAAGCTTATTAGAAGCCACAGCTGCCCTCC 3'.

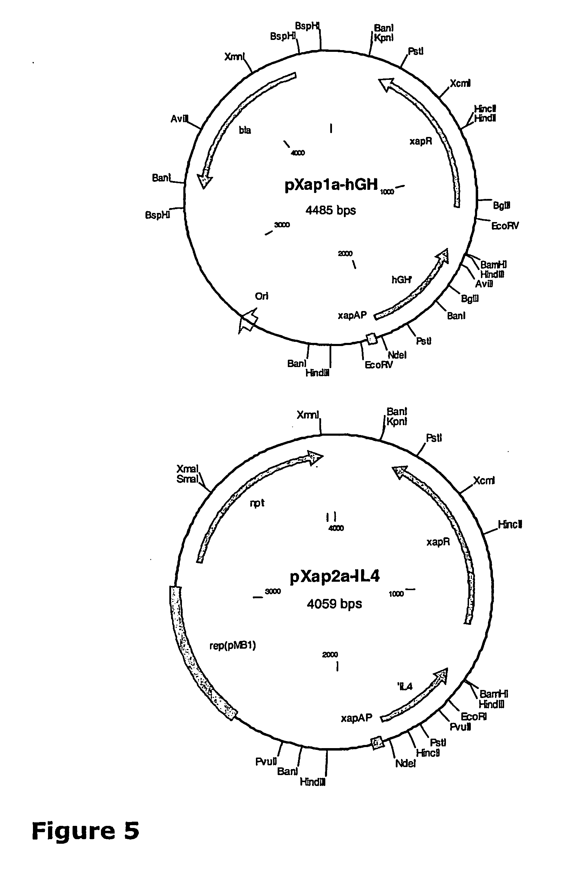

The amplification conditions and parameters were as described in example 1. The amplified fragment was cloned into pXap1a using the *Nde*I/*Bam*HI restriction sites (Figure 5). The production of human growth hormone by this new clone was tested in *E. coli* strain TG1, at 30°C in both complex (LB) and defined media (Miller 1992).

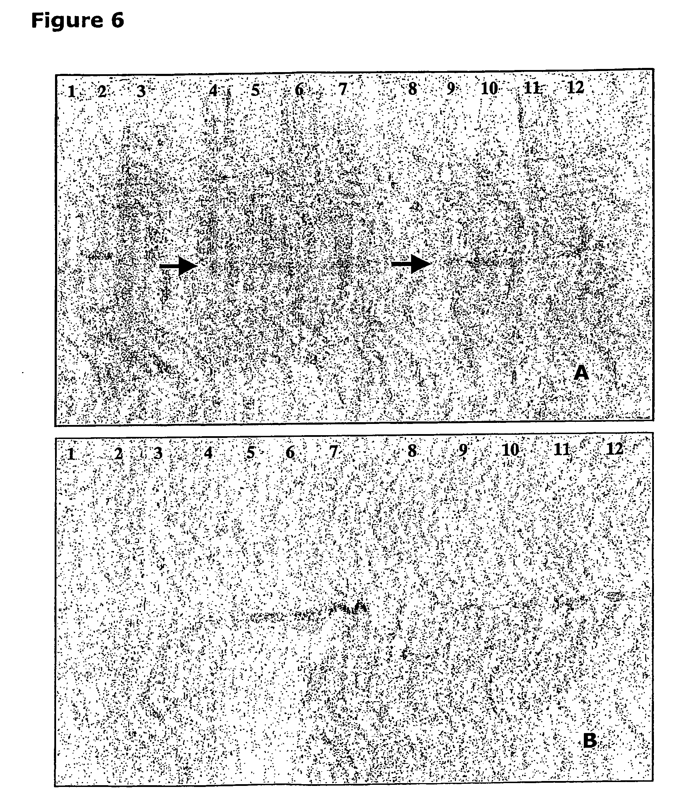

The expression of hGH was induced by addition of 1mg/ml of xanthosine to the cultures. The presence and identity of human growth hormone produced was confirmed by SDS-PAGE, and by Western-blot analysis, using a monoclonal antibody and detected using goat anti-mouse IgG-AP from BIO-RAD according to the manufacturers instructions (Figure 6). Densitometry analysis showed that the recombinant hGH protein represented greater than 20% of the total cell protein, even after only 2 hours induction, in either media.

### Expression of human interleukin-4

The Interleukin-4 gene (IL-4) was PCR amplified from a hIL-4 plasmid - pPlc299hIL4 (Dr. Nico Mertens, VIB, Belgium) using primers IL4-Nde (SEQ_ID10) 5' AAGAATCCCATATGCACAAGTGCGATATCACC 3' and IL4-Rev (SEQ_ID11) 5' AAGGATCCCAAGCTTAGCTCGAACACTTTGAATATTTC 3'.

The amplification conditions and parameters were as described before (Section: PCR amplification of the XapR gene). The amplified fragment was cloned into pXap2a using the *Nde*I/*Bam*HI restriction sites (Figure 5). The presence and identity of interleukin-4 was evaluated at 20°C and 28°C in LB medium. The expression of IL-4 was induced by addition of 1mg/ml of xanthosine to the cultures, and confirmed by SDS-PAGE and by Western-blot analysis, using a monoclonal antibody and detected using goat anti-mouse IgG-AP from BIO-RAD according to the manufacturer's instructions (Figure 7). Densitometry analysis showed that the recombinant IL-4 constituted over 30% of the total cell protein, at both temperatures.

### References

Bohannon, D and Soneshein, A (1989); J Bact **171**: 4718-27
Chen H, et al. (1994); Nuc Acid Res 22: 4953-7
Demain A, and Davies J (1999) "Manual of Industrial Microbiology and Biotechnology" "2nd Edition. ASM Press, Washington DC, USA.
Hammer-Jespersen, K et al. (1980); Mol Gen Genet 179: 341-8
Henikoff, S et al. (1988); PNAS 85: 6602-6
Janson, J-C and Rydén, L (1998) "Protein Purification: Principles, High-Resolution Methods, and Applications". John Wiley & Sons, NY, USA.
Jorgensen, C. and Dandanell, G., (1999); J. Bact. 181: 14, 4397-4403
Marquis D, et al. (1986); Gene 42: 175-83
Miller J.H. (1972); Experiments in Molecular Genetics. Cold Spring Harbor Laboratory, Cold Spring Harbor, New York.
Miller J. H. (1992); A Short Course in Bacterial Genetics. Cold Spring Harbor Laboratory, Cold Spring Harbor, New York.
Nygaard, P (1983); pp 27-93; Metabolism of nucleotides, nucleosides and nucleobases in microorganisms. Academic Press, London.
Seeger, C. et al. (1995); J Bact 177:19 5506-16

### Appendix 1 - DNA sequences

SEQ_ID1
   Primer Xap2
   ACGGTACCTTTTGCTATCTGCGATTTGCG
SEQ_ID2
   Primer Xap5
   CTCATTAAAAGGATCCGCGGCTCTGCTCTTCAG
SEQ_ID3
   Primer Xap1
   CCAAGCTTAGCATAATTCCCTATGCCGATC
SEQ_ID4
   Primer Xap4
   GAACCTGAGACATATGTATCCTTTTG
SEQ_ID5
   Primer xapNcoR
   GTGGTCACCATGGGTATCCTTTTTCTGTAGG
SEQ_ID6
   Primer B-Gal-1
   ATATGGGCCCATGGATCCCGTCGTTTTAC
SEQ_ID7
   Primer B-Gal-2
   AGTGTGAAGCTTATTATTTTTGACACCAG
SEQ_ID8
   Primer hGH-Nde
   AAGAATCCCATATGTTCCCAACCATTCCCTTATCC
SEQ_ID9
   Primer hGH-Rev
   CGCGGATCCAAGCTTATTAGAAGCCACAGCTGCCCTCC
SEQ_ID10
   Primer IL4-Nde
   AAGAATCCCATATGCACAAGTGCGATATCACC
SEQ_ID11
   Primer IL-4-Rev
   AAGGATCCCAAGCTTAGCTCGAACACTTTGAATATTTC
SEQ_ID12
   Primer pXapRBS1A
   CCTACAGAAAAAGGAGGTACCCATGGATCCCGTCG
SEQ_ID13
   Primer pXapRBS1B
   CGGGATCCATGGGTACCTCCTTTTTCTGTAGGGTGG
SEQ_ID14
   Primer pXapRBS2A
   CCCTACAGAAAAAGGAGATATCCCATGGATCCCGTCGTTTTACAACG
SEQ_ID15
   Primer pXapRBS2B
   CGACGGGATCCATGGGATATCTCCTTTTTCTGTAGGGTGGAATCTAACG

## Claims

1. A vector, comprising a promoter which can be operably linked to a gene encoding a heterologous protein, wherein the promoter induces expression of any operably linked heterologous protein, in the presence of nucleotides, which nucleotides may or may not have a phosphate group and wherein the promoter sequence, is a xapA promoter, which comprises ribosomal binding site having the following sequence:
AGGAGG xxxxx or
AGGAGG xxxxxx or
AGGAGA xxxxx or
AGGAGG xxxxxx

2. A vector, as claimed in claim 1, which is a plasmid or a bacteriophage.

3. A vector, as claimed in claim 1, which is an inducible expression vector.

4. A vector, as claimed in any one of claims 1 to 3, wherein the promoter induces expression of the operably linked heterologous protein in the presence of xanthosine.

5. A vector, as claimed in any one of claims 1 to 4, which further comprises a regulatory element which regulates expression by the promoter.

6. A vector, as claimed in claim 5, wherein the regulatory element is a nucleic acid sequence.

7. A vector, as claimed in claim 6, wherein the regulatory element is from a xapR gene.

8. A vector, as claimed in claim 6 or claim 7, wherein a further promoter is operably linked to the regulatory element.

9. A vector, as claimed in any one of claims 6 to 8, wherein the further promoter is an inducible or a constitutive promoter.

10. A vector, as claimed in claim 8, wherein the further promoter is from a xapR promoter.

11. A vector, as claimed in any one of claims 1 to 10, which comprises a gene encoding a heterologous protein.

12. A vector, as claimed in claim 11, wherein the gene encoding a heterologous protein encodes a cytokine, chemokine, hormone, enzyme or antigen,

13. An expression vector, comprising an isolated nucleic acid, comprising the regulatory xapR gene from the xanthosine operon together with a promoter from a xapA gene.

14. A xapA promoter sequence, which comprises a ribosomal binding site having the following sequence:
AGGAGG xxxxx or
AGGAGG xxxxxx or
AGGAGA xxxxx or
AGGAGA xxxxxx

15. A host cell, which comprises one or more vectors or nucleic acid sequences, as claimed in any one of claims 1 to 14.

16. A method for the expression of a heterologous protein, the method comprising culture of host cells, as claimed in claim 15, under conditions which induce the expression of the heterologous protein.

17. A method as claimed in claim 16, further comprising purification of the heterologous protein.

18. A method as claimed in claim 16 or claim 17, wherein the heterologous protein is a cytokine, chemokine, hormone, enzyme or antigen.

19. Use of one or more vectors or nucleic acid sequences, as claimed in any one of claims 1 to14 in the production of a heterologous protein.

## Patentansprüche

1. Vektor, welcher einen Promotor umfasst, der mit einem Gen, das für ein heterologes Protein codiert, funktionell verknüpft sein kann, wobei der Promotor die Expression von jeglichem funktionell verknüpften heterologen Protein in Anwesenheit von Nukleotiden induziert, welche Nukleotide eine Phosphatgruppe umfassen können oder auch nicht, und wobei die Promotorsequenz ein xapA-Promotor ist, welcher eine Ribosomen-Bindungsstelle mit der folgenden Sequenz umfasst:
AGGAGG xxxxx oder
AGGAGG xxxxxx oder
AGGAGA xxxxx oder
AGGAGA xxxxxx .

2. Vektor nach Anspruch 1, welcher ein Plasmid oder ein Bakteriophage ist.

3. Vektor nach Anspruch 1, welcher ein induzierbarer Expressionsvektor ist.

4. Vektor nach einem der Ansprüche 1 bis 3, wobei der Promotor die Expression des funktionell verknüpften heterologen Proteins in Anwesenheit von Xanthosin induziert.

5. Vektor nach einem der Ansprüche 1 bis 4, welcher weiters ein regulierendes Element umfasst, welches die Expression durch den Promotor reguliert.

6. Vektor nach Anspruch 5, wobei das regulierende Element eine Nukleinsäuresequenz ist.

7. Vektor nach Anspruch 6, wobei das regulierende Element von einem xapR-Gen stammt.

8. Vektor nach Anspruch 6 oder 7, wobei ein weiterer Promotor funktionell mit dem regulierenden Element verknüpft ist.

9. Vektor nach einem der Ansprüche 6 bis 8, wobei der weitere Promotor ein induzierbarer oder ein konstitutiver Promotor ist.

10. Vektor nach Anspruch 8, wobei der weitere Promotor von einem xapR-Promotor stammt.

11. Vektor nach einem der Ansprüche 1 bis 10, welcher ein für ein heterologes Protein codierendes Gen umfasst.

12. Vektor nach Anspruch 11, wobei das für ein heterologes Protein codierende Gen für ein Cytokin, Chemokin, Hormon, Enzym oder Antigen codiert.

13. Expressionsvektor, welcher eine isolierte Nukleinsäure umfasst, umfassend das regulierende xapR-Gen aus dem Xanthosin-Operon zusammen mit einem Promotor aus einem xapA-Gen.

14. xapA-Promotor-Sequenz, welche eine Ribosomenbindungsstelle mit der folgenden Sequenz aufweist:
AGGAGG xxxxx oder
AGGAGG xxxxxx oder
AGGAGA xxxxx oder
AGGAGA xxxxxx .

15. Wirtszelle, welche einen oder mehrere Vektoren oder Nukleinsäuresequenzen nach einem der Ansprüche 1 bis 14 umfasst.

16. Verfahren zur Expression eines heterologen Proteins, welches Verfahren das Züchten von Wirtszellen nach Anspruch 15 unter Bedingungen, die die Expression des heterologen Proteins induzieren, umfasst.

17. Verfahren nach Anspruch 16, welches weiters das Reinigen des heterologen Proteins umfasst.

18. Verfahren nach Anspruch 16 oder 17, wobei das heterologe Protein ein Cytokin, Chemokin, Hormon, Enzym oder Antigen ist.

19. Verwendung von einem oder mehreren Vektoren oder Nukleinsäuresequenzen nach einem der Ansprüche 1 bis 14 bei der Herstellung eines heterologen Proteins.

## Revendications

1. Vecteur comprenant un promoteur qui peut être relié de manière opérationnelle à un gène codant pour une protéine hétérologue, dans lequel le promoteur induit l'expression de n'importe quelle protéine hétérologue reliée de manière opérationnelle, en présence de nucléotides, lesquels nucléotides peuvent avoir ou non un groupement phosphate, et dans lequel la séquence de promoteur est un promoteur de xapA, qui comprend un site de liaison au ribosome ayant la séquence suivants :
AGGAGG xxxxx ou
AGGAGG xxxxxx ou
AGGAGA xxxxx ou
AGGAGA xxxxxx.

2. Vecteur selon la revendication 1, qui est un plasmide ou un bactériophage.

3. Vecteur selon la revendication 1, qui est un vecteur d'expression inductible.

4. Vecteur selon l'une quelconque des revendications 1 à 3, dans lequel le promoteur induit l'expression de la protéine hétérologue reliée de manière opérationnelle en présence de xanthosine.

5. Vecteur selon l'une quelconque des revendications 1 à 4, comprenant en outre un élément de régulation qui régule l'expression par le promoteur.

6. Vecteur selon la revendication 5, dans lequel l'élément de régulation est une séquence d'acide nucléique.

7. Vecteur selon la revendication 6, dans lequel l'élément de régulation provient d'un gène xapR.

8. Vecteur selon la revendication 6 ou 7, dans lequel un autre promoteur est relié de manière opérationnelle à l'élément de régulation.

9. Vecteur selon l'une quelconque des revendications 6 à 8, dans lequel l'autre promoteur est un promoteur inductible ou constitutif.

10. Vecteur selon la revendication 8, dans lequel l'autre promoteur provient d'un promoteur de xapR.

11. Vecteur selon l'une quelconque des revendications 1 à 10, comprenant un gène codant pour une protéine hétérologue.

12. Vecteur selon la revendication 11, dans lequel le gène codant pour une protéine hétérologue code pour une cytokine, une chimiokine, une hormone, une enzyme ou un antigène.

13. Vecteur d'expression comprenant un acide nucléique isolé, comprenant le gène de régulation xapR issu de l'opéron de la xanthosine conjointement avec un promoteur issu d'un gène xapA.

14. Séquence de promoteur de xapA, comprenant un site de liaison au ribosome, ayant la séquence suivants :
AGGAGG xxxxx ou
AGGAGG xxxxxx ou
AGGAGA xxxxx ou
AGGAGA xxxxxx.

15. Cellule hôte comprenant un ou plusieurs vecteurs ou séquences d'acide nucléique, selon l'une quelconque des revendications 1 à 14.

16. Procédé pour l'expression d'une protéine hétérologue, le procédé comprenant la culture de cellules hôtes selon la revendication 15, dans des conditions qui induisent l'expression de la protéine hétérologue.

17. Procédé selon la revendication 16, comprenant en outre la purification de la protéine hétérologue.

18. Procédé selon la revendication 16 ou 17, dans lequel la protéine hétérologue est une cytokine, une chimiokine, une hormone, une enzyme ou un antigène.

19. Utilisation d'un ou de plusieurs vecteurs ou d'une ou de plusieurs séquences d'acide nucléique selon l'une quelconque des revendications 1 à 14 dans la production d'une protéine hétérologue.
